(19) Europäisches Patentamt
European Patent Office
Office européen des brevets

(11) Veröffentlichungsnummer: **0 022 056**
**B1**

(12) # EUROPÄISCHE PATENTSCHRIFT

(45) Veröffentlichungstag der Patentschrift: **29.06.83**

(51) Int. Cl.³: **C 07 C 149/23,** C 07 C 163/00, C 07 F 7/10, A 61 K 49/04

(21) Anmeldenummer: **80730044.7**

(22) Anmeldetag: **27.06.80**

(54) **Dimere trijodierte Isophthalsäurediamide, deren Herstellung, diese enthaltendes Röntgenkontrastmittel, und dimere trijodierte Isophthalsäure-chloride.**

(30) Priorität: **28.06.79 DE 2926428**
**26.10.79 DE 2943777**
**02.05.80 DE 3017304**

(43) Veröffentlichungstag der Anmeldung:
**07.01.81 Patentblatt 81/1**

(45) Bekanntmachung des Hinweises auf die Patenterteilung:
**29.06.83 Patentblatt 83/26**

(84) Benannte Vertragsstaaten:
**AT BE CH DE FR GB IT LI LU NL SE**

(56) Entgegenhaltungen:
**DE-A-2 628 517**
**US-A-3 939 204**

(73) Patentinhaber: **SCHERING AKTIENGESELLSCHAFT**
**Berlin und Bergkamen,**
**Müllerstrasse 170/178 Postfach 65 03 11,**
**D-1000 Berlin 65 (DE)**

(72) Erfinder: **Mützel, Wolfgang, Dr., Weddigenweg 74,**
**D-1000 Berlin 45 (DE)**
Erfinder: **Siefert, Hans-Martin, Dr., Güntzelstrasse 17-18,**
**D-1000 Berlin 31 (DE)**
Erfinder: **Speck, Ulrich, Dr., Benediktiner Strasse 50,**
**D-1000 Berlin 28 (DE)**
Erfinder: **Pfeiffer, Heinrich, Dr., Pulfrichzeile 9,**
**D-1000 Berlin 20 (DE)**
Erfinder: **Schulze, Paul-Eberhard, Dr., Endestrasse 30,**
**D-1000 Berlin 39 (DE)**
Erfinder: **Acksteiner, Bernhard, Dr.,**
**Ludwigkirchstrasse 9a, D-1000 Berlin 15 (DE)**

EP 0 022 056 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Dimere trijodierte Isophthalsäurediamide, deren Herstellung, diese enthaltendes Röntgenkontrastmittel und dimere trijodierte Isophthalsäurechloride.

Gegenstand der Erfindung sind neue trijodierte 5-Aminoisophthalsäurederivate der allgemeinen Formel I

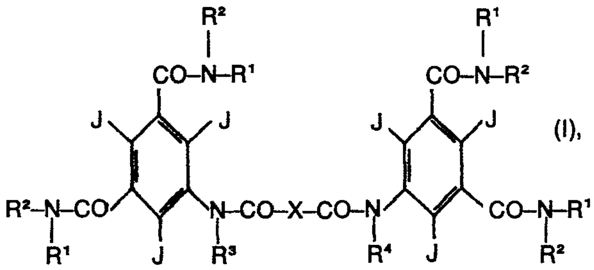

(I),

worin

$R^1$ einen gerad- oder verzweigtkettigen Mono- oder Polyhydroxyalkylrest mit 2–8 Kohlenstoffatomen und 1–5 Hydroxygruppen,

$R^2$ Wasserstoff, einen Alkylrest mit 1–4 Kohlenstoffatomen oder $R^1$,

$R^3$ und $R^4$ gleich oder verschieden sind und Wasserstoff oder einen Alkylrest mit 1–4 Kohlenstoffatomen bedeuten und

X ein gerad- oder verzweigtkettiges Alkylen mit 2–12 Kohlenstoffatomen, das durch 1 bis 3 Schwefel- oder 1 bis 2 Selenatome und gegebenenfalls zusätzlich durch ein Sauerstoffatom oder durch eine Di-($C_1$–$C_4$-alkyl)-silylgruppe oder eine Tetra-($C_1$–$C_4$-alkyl)-disiloxangruppe unterbrochen ist, darstellt und ein Verfahren zur Herstellung der Verbindungen der Formel I, Röntgenkontrastmittel, die Verbindungen der Formel I als schattengebende Substanz enthalten und die in Anspruch 7 genannten Verbindungen der Formel II.

Der Rest $R^1$ enthält als gerad- oder verzweigtkettiger niederer Mono- oder Polyhydroxyalkylrest 2–8 Kohlenstoffatome, vorzugsweise 2–5 Kohlenstoffatome. Geradkettige Reste von $R^1$ bestehen vorzugsweise aus 2–4 Kohlenstoffatomen, verzweigtkettige vorzugsweise aus 3–5, insbesondere 3 Kohlenstoffatomen. Die Hydroxygruppen im Rest $R^1$ können als primäre oder sekundäre Hydroxygruppen vorliegen. Der Rest $R^1$ kann 1–5 Hydroxygruppen enthalten, bevorzugt sind 1–3 und insbesondere 2-Hydroxygruppen. Als Reste $R^1$ seien beispielsweise genannt: 2-Hydroxyäthyl-, 2-Hydroxypropyl-, 3-Hydroxypropyl-, 3-Hydroxy-1-methyl-propyl-, 1-(Hydroxymethyl)-äthyl-, 3-Hydroxy-1-methyl-butyl-, 2-Hydroxy-isobutyl-, 2,3-Dihydroxypropyl-, 2,3-Dihydroxybutyl-, 2,4-Dihydroxybutyl-, 3,4-Dihydroxybutyl-, 3-Hydroxy-2-(hydroxymethyl)-propyl-, 2-Hydroxy-3-(hydroxymethyl)-propyl-, 2,3-Dihydroxy-1-methyl-propyl-, 2-Hydroxy-3-(hydroxymethyl)-butyl-, 1,3,4-Trihydroxybutyl-, 2,3,4-Trihydroxybutyl-, 2,4-Dihydroxy-3-(hydroxymethyl)-butyl-, 3-Hydroxy-2-bis(hydroxymethyl)-propyl-, 4-Hydroxy-3,3-bis-(hydroxymethyl)-butyl-, 4-Hydroxy-2,2-bis(hydroxymethyl)-butyl-, 2-Hydroxy-1,1-bis(hydroxymethyl)-äthyl-, $-CH_2-(CH{\cdot}OH)_4{\cdot}CH_2{\cdot}OH$.

Wenn $R^2$, $R^3$ und $R^4$ einen niederen Alkylrest bedeuten, sind damit insbesondere geradkettige Reste mit 1–4 Kohlenstoffatomen, bevorzugt 1–2 Kohlenstoffatomen gemeint wie beispielsweise Butyl, Propyl, Äthyl und insbesondere Methyl.

X als gerad- oder verzweigtkettiges Alkylen, das durch ein oder mehrere Schwefel- oder Selenatome und gegebenenfalls zusätzlich durch Sauerstoffatome oder durch eine Di-niederalkylsilyl-Gruppe oder eine Tetra-(nieder-alkyl)-disiloxan-Gruppe unterbrochen ist, kann 2–12, bevorzugt 2–8 Kohlenstoffatome enthalten. Insbesondere geeignet ist ein geradkettiges Alkylen mit 2–4 Kohlenstoffatomen, das durch ein oder mehrere, vorzugsweise durch ein, zwei oder drei Schwefel- oder ein oder zwei Selenatome oder durch eine Di-nieder-alkyl-silyl-Gruppe oder eine Tetra-(nieder-alkyl)-disiloxan-Gruppe unterbrochen ist. Als Nieder-alkyl-Substituenten kommen insbesondere geradkettige Alkylreste mit 1 bis 4, bevorzugt 1 bis 2 Kohlenstoffatomen in Frage; beispielsweise genannt seien der Butyl-, Propyl-, Äthyl- und insbesondere der Methylrest.

Ist das Alkylen X verzweigtkettig, ist der so gebildete Substituent ein niederer Alkylrest mit 1–4 Kohlenstoffatomen, insbesondere der Methyl- oder auch Äthylrest. Als Beispiele seien hier genannt

$-CH_2\cdot CH_2\cdot S\cdot CH_2\cdot CH_2-$, $-CH_2-S-S-CH_2-$,
$-CH_2\cdot CH_2-S-S-CH_2\cdot CH_2-$,
$-CH_2-S-CH_2\cdot CH_2-S-CH_2-$,
$-CH_2\cdot S-CH_2\cdot CH_2\cdot CH_2\cdot CH_2\cdot S\cdot CH_2-$,
$-CH_2-S-CH_2-S-CH_2$,
$-CH_2\cdot CH_2-S-CH_2-S-CH_2\cdot CH_2-$, 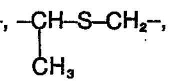
$-CH_2\cdot CH_2-Se-CH_2\cdot CH_2-$, $-CH_2\cdot S\cdot CH_2\cdot CH_2-$,
$-CH_2-S-CH_2\cdot CH_2\cdot CH_2-$,
$-CH_2\cdot CH_2-S-CH_2-CH_2-CH_2-$,
$-CH_2-O-CH_2-CH_2-S-CH_2-$, 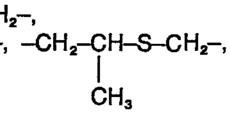

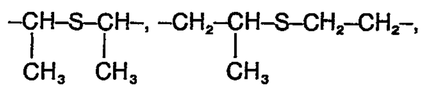

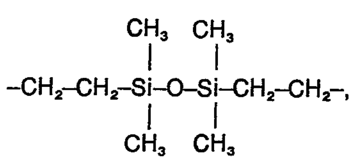

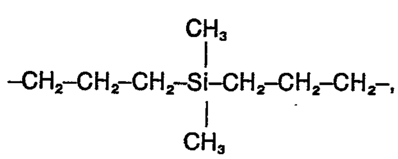

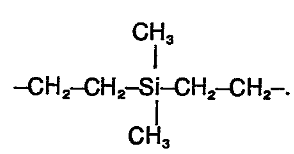

Für die Röntgendiagnostik z.B. der harnableitenden Organe und der angiographisch zu erfassenden Gefässe wurden als Kontrastmittel gut verträgliche Salze von 2,4,6-Trijodid-benzoesäuren entwickelt. Diese Substanzen werden vom Organismus bei höherer Dosierung jedoch nicht ohne Nebenwirkungen toleriert, obgleich ihre Toxizität oft gering ist. Eine ausreichende Darstellung des Gefässsystems, der ableitenden Harnwege, aber auch der cerebrospinalen Höhlen und anderer Systeme erfordert die Anwendung hoher Kontrastmittel-Dosierungen oder hochkonzentrierter Lösungen. Damit gewinnen die physikochemischen Eigenschaften der Kontrastmittel und deren Lösungen stark an Bedeutung, da wesentliche pharmakologische Effekte wie Schmerz, Blutdruckabfall, Gefässschädigung und viele andere auf diese zurückgeführt werden müssen.

Mit der Entwicklung der Dimeren durch Verknüpfung von zweitrijodierten Benzoesäuren konnten gegenüber den monomeren trijodierten Benzoesäuren unter anderem die neurale Verträglichkeit verbessert und die Vasolidation in der Angiographie vermindert werden. Durch den etwas geringeren osmotischen Druck zum Beispiel der dimeren locarminsäure (als Dimegluminsalz) erhöht sich dessen Konzentration im Harn.

Dimere ionische, mit radioaktivem Jod kernsubstituierte Verbindungen dieser Art werden in der US-Patentschrift 3 939 204 beschrieben; sie können als radioaktive Funktionsdiagnostika verwendet werden.

Nichtionische dimere hexajodierte Verbindungen, deren Brückenalkylen durch Sauerstoffatome unterbrochen sein können, werden in der DAS 26 828 517 beschrieben.

Mit der Einführung des nichtionischen Metrizamids gelang es erstmals bei einem monomeren trijodierten Kontrastmittel, den osmotischen Druck bedeutend zu senken. Auch beim Metrizamid blieb der osmotische Druck einer beispielsweise für die Angiographie geeigneten Lösung (300 mg Jod/ml) mit 12 atm noch beträchtlich über dem osmotischen Druck des Blutes (7,5 atm). Seine Eignung in der Myelographie, Angiographie und Urographie wurde in mehreren Arbeiten der Acta Radiol. Suppl. 335 (1973) beschrieben. Dagegen sind alle bisher beschriebenen wasserlöslichen Kontrastmittel, ebenso wie das Metrizamid, nicht für die Lymphographie geeignet.

Eine wichtige Rolle spielen die physikalischen Eigenschaften auch bei einem Röntgenkontrastmittel für die Anwendung in der Lymphographie.

In der medizinischen Praxis werden für die Lymphographie zur Zeit überwiegend jodierte Öle, z.B. jodierte Fettsäureester des Mohnöls, verwendet. Diese Mittel zeigen einerseits zwar eine gute Speicherfähigkeit in den Lymphknoten und gute Kontrastwirkung, sind aber anderseits aufgrund ihrer bekannten Nebenwirkungen weder allgemein noch problemlos anwendbar. Sie können im Hinblick auf die leichte Verletzlichkeit der Lymphgefässe, insbesondere bei zu schneller Applikation, Lymphgefässläsionen begünstigen. Eine Reihe von Nachteilen sind im Ölcharakter dieser Kontrastmittel begründet: da die Suspendierung des Öls erst im Blut erfolgt, ist die Öltröpfchengrösse nicht im voraus bestimmbar. Fallen die Öltröpfchen zu gross aus, sind häufig Ölmikroembolien, z.B. in der Lunge, unvermeidbar. Da die Aufnahme der grossen Öltröpfchen aus den interstitiellen Räumen in die Lymphkapillaren kaum möglich ist, gelingt eine Darstellung der Lymphbahnen und Lymphknoten beispielsweise nach subkutaner oder intraparenchymatöser Injektion nur in Ausnahmefällen.

Letzterer Nachteil ist nur durch endolymphatische Applikation zu überwinden, wobei jedoch eine ebenfalls nicht komplikationslose vorherige Farbstoffmarkierung und operative Freilegung der Lymphgefässe unter Lokalanästhesie in Kauf genommen werden muss. Schliesslich ist nicht unbeachtlich, dass die Kontrastmittel auf Basis jodierter Öle nach erfolgter Applikation nur sehr langsam wieder ausgeschieden werden. In Abhängigkeit von der Zubereitungsform beträgt die Ausscheidungszeit Wochen bis Monate.

Die aufgezeigten Nachteile hat man versucht, durch Emulgieren dieser jodierten Öle zu überwinden. Dadurch wurde zwar erreicht, die Viskosität und Tröpfchengrösse zu verringern, wodurch die Kapillarpassage verbessert und bei Stabilität der Emulsion das Embolisationsrisiko gesenkt werden konnte. Diese Teilerfolge konnten jedoch nur durch andere Nachteile erkauft werden. Beispielsweise genannt seien in diesem Zusammenhang: effektive Reduktion des Jodgehaltes pro ml Kontrastmittel und damit verbunden unausbleiblicher Kontrastverlust, grössere Lokaltoxizität am Lymphknoten, hepatotoxische Effekte und histologisch nachweisbare Fremdkörperreaktion, verstärkt durch beigefügte Emulgatoren.

Alle Kontrastmittel auf Basis jodierter Öle sind wenig stabil und aufgrund der dargestellten Nebenwirkungen nur begrenzt anwendbar.

Die bekannten wasserlöslichen organischen Jodverbindungen in den üblichen Zubereitungen für Uro- und Angiographie erwiesen sich ebenfalls als wenig bis gar nicht geeignet für die Lymphographie, weil sie nach Applikation zu schnell wieder aus dem Lymphgefässsystem hinausdiffundieren. Auf dieser mangelhaften Speicherfähigkeit in den Lymphknoten sind diese wässrigen Zubereitungsformen bestenfalls begrenzt geeignet für die periphere Lymphangiographie.

Auch Versuche mit Kristallsuspensionen auf Basis kernjodierter Aromaten, wie z.B. mit Jodamid oder Tetraiodterephthalsäurederivaten, führten aufgrund ihrer zu geringen Verträglichkeit (Entzündungseffekte an der Injektionsstelle und im Lymphsystem) und ihrer zu langen Ausscheidungszeit (Tage bis Wochen) nicht zu in der medizinischen Praxis brauchbaren Kontrastmitteln für die Lymphographie.

Zusammenfassend ist festzustellen, dass die Lymphographie mit diesen bekannten Röntgenkontrastmitteln nicht ohne Risiko möglich und deshalb nur stationär durchführbar ist, wobei der Patient sehr sorgfältig auf seine Eignung für diese Untersuchung getestet werden muss. Für die

Durchführung einer solchen Untersuchung werden ab Eignungstest bis zur eigentlichen Lymphographie mindestens drei Tage benötigt.

Der vorliegenden Erfindung lag die Aufgabe zugrunde, neue schattengebende Substanzen auf Basis kernjodierter Aromaten und Röntgenkontrastmittel zu entwickeln, die zur Darstellung von Körperhohlräumen allgemein und insbesondere auch für die Lymphographie anwendbar sind.

Es wurde gefunden, dass die erfindungsgemässen Verbindungen der allgemeinen Formel I in hervorragender Weise als schattengebende Substanzen in Röntgenkontrastmitteln geeignet sind. Überraschenderweise ist die wässrige Lösung von Verbindungen der Formel I auch bei der direkten und indirekten Lymphographie anwendbar.

Unter indirekter Lymphographie versteht man alle Methoden, bei denen schattengebende Substanzen z.B. in die Haut und Unterhaut, Schleimhäute, parenchymatöse und nichtparenchymatöse Organe, seröse und kavitäre Höhlen, Muskeln und Knorpel und Knochen appliziert werden. Die Verbindungen der Formel I sind sehr gut wasserlöslich, ohne mit dem Nachteil einer verstärkten Diffusion durch die Lymphgefässwände belastet zu sein. Die erhaltenen Lymphogramme sind somit ausgezeichnet durch eine scharfe Begrenzung gegenüber der Umgebung.

Die Röntgenkontrastmittelzubereitungen auf Basis der erfindungsgemässen Verbindungen sind sehr beständig, sind weder luft- noch lichtempfindlich und zeigen weder in Substanz noch in Lösung eine unerwünschte Jodabspaltung.

Bezüglich der Verträglichkeit ist von besonderem Vorteil, dass sie nicht mit dem Embolisationsrisiko belastet sind.

Die Speicherfähigkeit der erfindungsgemässen Kontrastmittel im Lymphgefässsystem sowohl nach direkter als auch indirekter Applikation ist gut. Bereits nach 5 bis 20 Minuten ist der Verlauf der Lymphangien und die Lymphknoten optimal kontrastiert.

Das Kontrastmittel verbleibt nach der Applikation mindestens 45 Minuten im Lymphsystem und wird innerhalb von etwa 24 Stunden praktisch vollständig über die Niere ausgeschieden. Da mit Verwendung der erfindungsgemässen Röntgenkontrastmittel in der Lymphographie der bisher notwendige Eignungstest nicht erforderlich ist, kann bei Verwendung dieser Mittel das erwünschte Lymphogramm ohne stationäre Behandlung erstellt werden, wodurch sich die Behandlungsdauer auf maximal 1½ bis 4 Stunden verkürzt.

Ein weiterer sehr wesentlicher Vorteil der erfindungsgemässen Röntgenkontrastmittel auf Basis der Verbindungen gemäss Formel I ist die indirekte Darstellbarkeit des Lymphsystems mindestens bis zur ersten, zum Teil aber auch bis zur zweiten Lymphknotenstation und darüber hinaus, wodurch die Früherkennung krankhafter Veränderungen, zum Beispiel eines metastatischen Befalls, und deren rechtzeitige Bekämpfung ermöglicht wird. Darüber hinaus besitzen die erfindungsgemässen Verbindungen in wässriger Lösung eine für Röntgenkontrastmittel erwünscht niedrige Viskosität, die die Verwendung kleinerer Kanülen bei der Anwendung dieser Röntgenkontrastmittel ermöglicht.

In der nachfolgenden Tabelle ist die vorteilhafte Wirkung der erfindungsgemässen Verbindungen in der Lymphographie am Beispiel von Thiodipropionsäure-bis-[3,5-bis(2,3-dihydroxypropyl-N-methyl-carbamoyl)-2,4,6-trijod-anilid] (A) und von 4,4,6,6-Tetramethyl-4,6-disila-5-oxanonandisäure-bis[3,5-bis(2,3-dihydroxypropyl-N-methyl-carbamoyl)-2,4,6-trijodid-anilid] (B) im Vergleich zum Fettsäureäthylester des jodierten Oleum Papaveris (C) gegenübergestellt. Die Testergebnisse wurden am Hund mit in der Lymphographie üblichen Testmethoden nach direkter und indirekter Applikation ermittelt.

Tabelle

| Bewertungsparameter | C | A + B |
|---|---|---|
| Anwendungsmöglichkeit | direkte Lymphographie | direkte und indirekte Lymphographie |
| Beständigkeit | licht-luftempfindlich (J-Abspaltung) | stabil |
| Verträglichkeit | Embolie-Risiko | keine Embolien |
| Verweilzeit im Körper | Wochen bis Monate | Stunden |
| Darstellungsbereich bei indirekter Lymphographie | keine Darstellung des Lymphsystems | Darstellung des Lymphsystems bis mindestens der ersten Lymphknotenstation |
| Behandlungszeit | mindestens 3 Tage | maximal 4 Stunden |

Die erfindungsgemässen Verbindungen sind aber nicht nur für die Lymphographie geeignet. Aufgrund ihrer physikalischen Eigenschaften, wie z.B. hohe Wasserlöslichkeit und niedriger osmotischer Druck, und pharmakologischen Eigenschaften, wie zum Beispiel extrem geringe diuretische Wirkung, sind sie als schattengebende Substanzen auch auf verschiedenen Anwendungsgebieten von wasserlöslichen Röntgenkontrastmitteln, insbesondere zur Darstellung von Körperhöhlen und auch z.B. der ableitenden Harnwege inklusive der retrograden Urographie, des Magen-Darm-Kanals, der Gelenkhöhlen, des cerebrospinalen und tracheobronchialen Systems, zur Hysterosalpingographie zur Gastroenteroskopie und zur Bronchoskopie, hervorragend geeignet. In allen Fällen kommt es durch die neuen Röntgenkontrastmittel zu einer besonders guten Detail-erkennbarkeit der dargestellten Strukturen.

Die Erfindung betrifft somit auch neue Röntgenkontrastmittel, die mindestens eine der erfindungsgemässen Verbindungen der Formel I enthalten.

Die Herstellung der neuen Röntgenkontrastmittel auf Basis der erfindungsgemässen Verbindungen der allgemeinen Formel I erfolgt in an sich bekannter Weise, z.B. dadurch, dass man die schattengebende Substanz mit den in der Galenik üblichen Zusätzen, z.B. Stabilisatoren wie Natriumedetat, Calcium-di-natriumedetat, physiologisch verträgliche Puffer, Natriumchlorid, Propylenglykol, Äthanol u.ä., in eine für die Applikation geeignete Form bringt. Die Konzentration der neuen Röntgenkontrastmittel im wässrigen Medium richtet sich ganz nach der röntgendiagnostischen Methode. Die bevorzugten Konzentrationen und Dosierungen der neuen Verbindungen bewegen sich in den Bereichen von 20–400 mg J/ml für die Konzentration und 5–500 ml für die Dosierung. Besonders bevorzugt sind Konzentrationen zwischen 100–400 mg J/ml.

Sollen die erfindungsgemässen Verbindungen speziell für die Lymphographie verwendet werden, erfolgt die Herstellung der wässrigen Kontrastmittellösungen z.B. in der Weise, dass man die entsprechende Wirksubstanz bei Raumtemperatur in Wasser zur Injektion einrührt, bis die Substanz vollständig gelöst ist. Durch Zugabe von Alkoholen, Basen, Säuren oder den üblichen Puffersystemen können die Eigenschaften der Lösung in der gewünschten Form beeinflusst werden.

Für dieses Anwendungsgebiet bewegen sich die bevorzugten Konzentrationen und Dosierungen in den Bereichen 200–400 mg J/ml für die Konzentration und 1–50 ml für die Dosierung.

Schliesslich betrifft die vorliegende Erfindung auch ein Verfahren zur Herstellung von trijodierten 5-Aminoisophthalsäurederivaten der allgemeinen Formel I, dadurch gekennzeichnet, dass man in an sich bekannter Weise ein Tetracarbonsäuretetrachlorid der allgemeinen Formel II

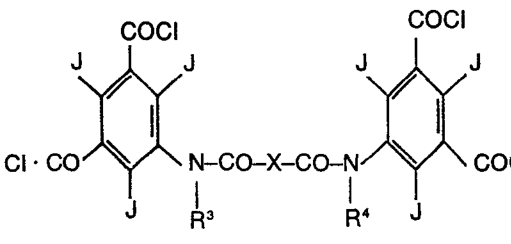

(II),

worin $R^3$, $R^4$ und X die obengenannte Bedeutung haben, mit einem Amin

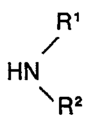

,

worin $R^1$ und $R^2$ die obengenannte Bedeutung haben, umsetzt.

Die Amidierung der CO·Cl-Gruppen wird in einem geeigneten, zweckmässigerweise polaren Lösungsmittel bei 0–100 °C, vorzugsweise bei 20–80 °C, durchgeführt. Als geeignete Lösungsmittel seien beispielsweise genannt Dioxan, Tetrahydrofuran, Dimethylformamid, Dimethylacetamid, Hexametapol u.ä. und deren Gemische. Da die Amidierungsreaktion exotherm verläuft, ist es gegebenenfalls zweckmässig, das Reaktionsgemisch leicht zu kühlen, um die Reaktionstemperatur unter 60 °C halten zu können.

Der während der Amidierungsreaktion freiwerdende Chlorwasserstoff wird entweder durch einen entsprechenden Überschuss an Base

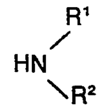

oder durch Zusatz eines üblichen Protonenakzeptors gebunden.

Als Protonenakzeptoren zur Neutralisation des bei der Amidierung entstehenden Chlorwasserstoffs verwendet man vorteilhaft tertiäre Amine, wie z.B. Triäthylamin oder Tributylamin.

Die im Reaktionsverlauf anfallenden anorganischen oder organischen Salze werden in bekannter Weise abgetrennt, vorteilhaft z.B. mit Hilfe üblicher Ionenaustauschersäulen oder durch Filtration über bekannte Adsorbentien, wie z.B. Diaion oder Amberlite® XAD-2 und 4.

Die verfahrensgemäss eingesetzten neuen Tetracarbonsäuretetrachloride der Formel II erhält man nach an sich bekannten Methoden aus dem bekannten 5-Amino- bzw. 5-nieder-Alkylamino-2,4,6-trijodisophthalsäuredichlorid durch Kondensation mit dem Dichlorid einer aliphatischen Dicarbonsäure der Formel Cl-CO-X-CO-Cl, worin X die obengenannte Bedeutung hat. Die Umsetzung erfolgt vorzugsweise bei über 100 °C. Als Reaktionsmedium sind organische Lösungsmittel, z.B. aromatische Kohlenwasserstoffe wie Chlorbenzol und Toluol, insbesondere aber inerte polare Lösungsmittel wie Dimethylacetamid, N-Methylpyrrolidon, Dioxan, Tetrahydrofuran u.a. geeignet. Die bei der Umsetzung gebildeten dimeren Tetracarbonsäuretetrachloride der allgemeinen Formel II kristallisieren entweder aus oder werden durch Einengen der Lösungen im Vakuum isoliert.

Sind im Tetracarbonsäuretetrachlorid der Formel II die Substituenten $R_3$ und $R_4$ verschieden ($R_3$=H, $R_4$ = niederes Alkyl, vorzugsweise Methyl), erfolgt die Umsetzung stufenweise, indem man in an sich bekannter Weise, z.B. wie oben beschrieben, 5-nieder-Alkyl-amino-2,4,6-trijodisophthalsäuredichlorid mit $Cl{\cdot}CO{-}X{-}COOCH_3$ (X hat die oben angegebene Bedeutung) umsetzt, den entstandenen Halbester in üblicher Weise verseift und die Trisäure in Form ihres Natriumsalzes isoliert. Das Na-Salz wird anschliessend mit z.B. Phosphorpentachlorid in üblicher Weise in das entsprechende Tricarbonsäure-tri-chlorid

übergeführt, das schliesslich mit 5-Amino-2,4,6-trijod-isophthalsäuredichlorid nach an sich bekannten Methoden, z.B. wie oben beschrieben, zum letztlich gewünschten Tetracarbonsäuretetrachlorid der Formel II, in dem $R_3$ und $R_4$ verschieden sind und ein niederes Alkyl (vorzugsweise Methyl) bzw. ein Wasserstoff bedeuten, kondensiert wird.

Die Dicarbonsäuredichloride der Formel Cl-CO-X-COCl bzw. der Halbester Cl-CO-X-CO·$OCH_3$, worin X ein gerad- oder verzweigtkettiges Alkylen bedeutet, das durch ein oder mehrere Schwefel- oder Selenatome und gegebenenfalls zusätzlich durch Sauerstoffatome unterbrochen ist, sind an sich bekannt. Die Herstellung der Vorstufen dieses Verbindungstyps erfolgt somit nach bekannten Methoden, wie sie beispielsweise in der offengelegten deutschen Patentanmeldung DOS 2 028 556 und von David K. Laing et al., J. Chem. Soc. Dalton Trans. 1975 (21) 2297 beschrieben sind. Die Herstellung der Dicarbonsäuredichloride der Formel ClCO-X-COCl bzw. der Halbesterchloride der Formel ClCOX-$COOCH_3$, worin X durch eine Di-niederalkyl-silyl-gruppe oder eine Tetra-niederalkyl-disiloxan-gruppe unterbrochen ist, kann man von den Dicarbonsäure-methylestern $CH_3OCO$-X-$COOCH_3$ ausgehen, wie sie beispielsweise von R.A. Benkeser et al. (J. Org. Chem. 32 [1967], 395) beschrieben sind, wobei man nach Methoden, die dem Fachmann bekannt sind, durch partielle Verseifung mit nur einem Äquivalent Base die Halbester $CH_3OCO$-X-COOH oder durch vollständige Verseifung mit einem Überschuss an Base bzw. durch Umesterung in alkoholischer Lösung in Gegenwart von Mineralsäure die Dicarbonsäuren HOOC-X-COOH erhält. Zur Herstellung der Halbester $CH_3$-OCO-X-COCl aus den Dicarbonsäuren HOOC-X-COOH, wie sie beispielsweise von L.H. Sommer et al. (J. Amer. Chem. Soc. 75 [1953], 2932) beschrieben sind, überführt man diese zunächst in die Dicarbonsäurediester, aus denen man dann – wie oben beschrieben – durch partielle Verseifung die Halbester $CH_3OCO$-X-COOH erhält. Durch Umsetzung mit Thionylchlorid oder Oxalylchlorid stellt man dann in an sich bekannter Weise aus den Halbestern die Halbesterchloride bzw. aus den Dicarbonsäuren die Dicarbonsäuredichloride her, wie am Beispiel der Herstellung von 4,4,6,6-Tetramethyl-4,6-disila-5-oxa-nonandisäure-dichlorid im einzelnen erläutert werden soll:

Zu 8,2 g 4,4,6,6-Tetramethyl-4,6-disila-5-oxa-nonandisäure destilliert man 40 ml Oxalylchlorid, wobei die Reaktion durch Eiskühlung gemildert wird. Anschliessend rührt man noch 45 Minuten bei Raumtemperatur und destilliert das überschüssige Oxalylchlorid im Vakuum ab. Der Rückstand wird im Vakuum fraktioniert, man erhält 6,2 g 4,4,6,6-Tetramethyl-4,6-disila-5-oxa-nonandisäure-dichlorid: $Kp_1$: 45 °C (66% der Theorie).

Die nachfolgenden Ausführungsbeispiele dienen der weiteren Erläuterung der vorliegenden Erfindung.

Beispiel 1

Thiodipropionsäure-bis[3,5-bis(2,3-Dihydroxypropyl-N- methyl-carbamoyl)-2,4,6-trijod-anilid]

A) Thiodipropionsäure-bis(3,5-chlorcarbonyl)-2,4,6-trijod-anilid

595,7 g 5-Amino-2,4,6-trijod-isophthalsäuredichlorid werden in 600 ml Dioxan heiss gelöst. Zu der siedenden Lösung werden unter Rühren innerhalb von 20 Minuten 97,3 ml Thiodipropionsäuredichlorid getropft. Anschliessend wird das Reaktionsgemisch 4 Stunden unter Rückfluss (Badtemp. 120–130 °C) gerührt. Nach etwa 1 Stunde fällt ein Niederschlag aus. Nach Rühren über Nacht bei Raumtemperatur wird der Niederschlag abgesaugt und bei 50 °C im Vakuum über Ätznatron ohne Luftstrom getrocknet. Rohausbeute: 543 g (81,4% der Theorie). Das rohe «Tetrachlorid» wird nochmals 30 Minuten mit 750 ml Dioxan auf dem Dampfbad ausgerührt, nach Abkühlen auf Raumtemperatur abgesaugt, mit wenig Dioxan gewaschen und bei 50 °C im Vakuum über Ätznatron ohne Luftstrom getrocknet. Ausbeute: 435 g (61% der Theorie) mit 6,5% Dioxan, Schmelzpunkt 253–254 °C (Zersetzung).

B) Amidierung

285 g Thiodipropionsäure-bis-(3,5-chlorcarbonyl)-2,4,6-trijod-anilid werden in 500 ml Dimethylacetamid gelöst und auf 50 °C erwärmt. Bei 50 °C werden innerhalb 45 Minuten unter Rühren 105 g N-Methylamino-propandiol-(2,3) gelöst in 300 ml Dimethylacetamid zugetropft, wobei eine Wärmetönung bis +58 °C auftritt. Es werden 237,6 ml Tributylamin zugegeben, dann wird 4 Stunden bei ca. 50 °C und über Nacht bei Raumtemperatur gerührt. Das Reaktionsgemisch wird dann mit 40 ml konzentrierter Salzsäure bis zur sauren Reaktion versetzt und tropfenweise in 4 l Methylenchlorid eingerührt. Nach 30 Minuten Rühren wird der Niederschlag abgesaugt, nochmals 30 Minuten mit 1,5 Liter Methylenchlorid ausgerührt, abgesaugt und bei 40 °C im Vakuum getrocknet.

Rohausbeute: 364 g.

200 g Rohprodukt (pH 4–5) werden in 2 Liter Methanol warm gelöst, filtriert und über einen schwach basischen Anionen-Austauscher, z.B. Levatit MP 7080, ca. 5 Liter gegeben. Tropfgeschwindigkeit ca. 1 Liter/h. Die Lösung fliesst mit ca. pH 10 ab. Am Rotavapor wird das Filtrat auf 2 Liter eingeeingt. Die erhaltene methanolische Lösung wird dann über einen schwach sauren Kationen-Austauscher, z.B. Levatit CP 3050, 5 Liter, gegeben. Die Lösung fliesst farblos mit pH > 6 ab. Die erhaltene methanolische Lösung wird auf 1,2 Liter eingeengt. In der Siedehitze werden dann 2,4 Liter i-Propanol zugetropft. Dabei bildet sich eine ölige Abscheidung, die beim Abkühlen erstarrt. Nach Rühren über Nacht wird der Niederschlag abgesaugt, mit wenig i-Propanol kalt gewaschen und mit Äthanol ausgerührt. Der feste Rückstand wird abgesaugt. Nach Trocknen im Vakuum bei 100 °C erhält man 100 g Thiodipropionsäure-bis-[3,5-bis-(2,3-Dihydroxypropyl-N-methyl-carbamoyl)-2,4,6-trijod-anilid]; Fp. 240–260 °C (Zersetzung).

Beispiel 2

20 g Thiodipropionsäure-bis-[3,5-bis(chlorcarbonyl)-2,4,6-trijodanilid] werden in 50 ml Dimethylacetamid gelöst und auf 50 °C erwärmt. Bei 50 °C werden innerhalb von 30 Minuten unter Rühren 6,8 g 1-Amino-propandiol-2,3 zugetropft, wobei leichte Wärmetönung auftritt. Es werden 17,8 ml Tributylamin zugegeben und 4 Stunden bei 50 °C und über Nacht bei Raumtemperatur gerührt. Das Reaktionsgemisch wird dann mit konzentrierter Salzsäure angesäuert und tropfenweise in 300 ml Methylenchlorid eingerührt. Nach Absaugen und Waschen des Niederschlags mit Methylenchlorid wird im Vakuum bei 40 °C getrocknet. Das Rohprodukt wird über einen in Dimethylacetamid aufbereiteten schwach basischen Anionen- und nachfolgend schwach sauren Kationenaustauscher gegeben. Man erhält eine fast farblose Lösung, die auf 200 ml eingeengt wird. Es werden nun etwa 800 ml Isopropanol zugegeben. Der erhaltene Niederschlag wird abgesaugt, mit Isopropanol gewaschen und mit wenig Wasser ausgerührt. Der Niederschlag wird bei 50 °C im Vakuum 10 Stunden getrocknet. Man erhält 17,5 g (75% der Theorie) Thiodipropionsäure-bis-[3,5-bis(2,3-dihydroxypropyl-carbamoyl)-2,4,6-trijod-anilid]; Fp. 300 °C (Zersetzung).

Beispiel 3

20 g Thiodipropionsäure-bis-[3,5-bis(chlorcarbonyl)-2,4,6-trijodanilid] werden in ein 50 °C heisses Gemisch von 200 ml Dioxan und 42 g N-Methylpropandiol-(1,3) langsam eingetragen. Das feste Tetrachlorid löst sich unter Wärmetönung schnell auf. Man rührt 2 Stunden bei 50 °C nach. In die Suspension werden nach Abkühlen 200 ml Dichlormethan eingerührt. Man dekantiert von der öligen Abscheidung und nimmt mit 50 ml Methanol auf. Die methanolische Lösung lässt man in 50 ml Isopropanol einfliessen und saugt den Niederschlag ab. Zur weiteren Aufreinigung wird das Rohprodukt in einem Gemisch Methanol/Dimethylacetamid gelöst und, wie in Beispiel 1 beschrieben, gereinigt. Man erhält 19,2 g (82% der Theorie) Thiodipropionsäure-bis-[3,5-bis-(1,3-dihydroxypropyl-N-methyl-carbamoyl)-2,4,6-trijod-anilid]; Fp. 300 °C (Zersetzung).

Beispiel 4

59,5 g 5-Amino-2,4,6-trijod-isophthalsäuredichlorid werden mit 12 g Dithiodiessigsäuredichlorid, wie in Beispiel 1 unter A) beschrieben, kondensiert. Man erhält 34,8 g (52% der Theorie) Dithiodiessigsäure-bis-[3,5-bis(chlorcarbonyl)-2,4,6-trijodanilid]; Fp. 285 °C (Zersetzung). 20 g dieses Tetrachlorids werden, wie in Beispiel 1 unter B) beschrieben, mit 7,9 g N-Methyl-amino-propandiol-(2,3) umgesetzt. Nach Aufarbeiten durch Fällen in Methylenchlorid, Entfernen der Anionen und Kationen mittels schwach basischen und schwach sauren Ionenaustauschern und Umfällen aus Methanol/Isopropanol (1:2) erhält man 12 g (60% der Theorie) Dithiodiessigsäure-bis-[3,5-bis-(2,3-dihydroxypropyl-N-methyl-carbamoyl)-2,4,6-trijod-anilid], Fp. 235 °C (Zersetzung).

Beispiel 5

59,5 g 5-Amino-2,4,6-trijod-isophthalsäure-dichlorid werden mit 13,5 g Bis-(2-chlorcarbonyläthyl)-disulfid, wie in Beispiel 1 unter A) beschrieben, kondensiert. Man erhält 42,2 g (62% der Theorie) 4,5-Dithiaoctan-disäure-bis-[3,5-bis-(chlorcarbonyl)-2,4,6-trijod-anilid]; Fp. 252 °C. 40 g dieses Tetrachlorids werden, wie in Beispiel 1 unter B) beschrieben, mit 15,2 g N-Methylamino-propandiol-(2,3) umgesetzt. Nach Aufarbeiten durch Fällen in Methylenchlorid, Entfernen der Anionen und Kationen mittels schwach basischen und schwach sauren Ionenaustauschern und Umfällen aus Methanol/Isopropanol (1:2) erhält man 30 g (63% der Theorie) 4,5-Dithiaoctandisäure-bis[3,5-bis-(2,3-dihydroxypropyl-N-methyl-carbaamoyl)-2,4,6-trijod-anilid]; Fp. 290–292,5 °C (Zersetzung).

Beispiel 6

59,5 g 5-Amino-2,4,6-trijod-isophthalsäure-dichlorid werden mit 11 g 2-Methyl-3-thiaglutarsäuredichlorid, wie in Beispiel 1 unter A) beschrieben, kondensiert. Man erhält ein Gemisch aus 5,5′-(2-Methyl-3-thiaglutaryldiimino)-bis-(2,4,6-trijod-isophthalsäuredichlorid) und 5-(2-Methyl-3,5-dioxoperhydro-1,4-thiazin-4-yl)-2,4,6-trijod-isophthalsäuredichlorid und trennt durch Chromatographie an Silicagel. Mit Chloroform als Elutionsmittel wird zuerst 5-(2-Methyl-3,5-dioxoperhydro-1,4thiazin-4-yl)-2,4,6-trijod-isophthalsäuredichlorid abgetrennt und dann 5,5′-(2-Methyl-3-thia-glutaryldiimino)-bis-(2,4,6-trijod-isophthalsäure-dichlorid) als Reinsubstanz gewonnen. Die Ausbeute beträgt 15,5 g (24% der Theorie); Fp. 255 °C (Zersetzung). 10 g dieses Tetrachlorids werden, wie in Beispiel 1 unter B) beschrieben, mit 4 g N-Methylamino-propandiol-(2,3) umgesetzt. Nach Aufarbeiten durch Fällen in Methylenchlorid, Entfernen der Anionen und Kationen mittels schwach basischen und schwach sauren Ionenaustauschern und Umfällen aus Methanol/Isopropanol (1:2) erhält man 6 g (50% der Theorie) 5,5′-(2-Methyl-3-thia-glutaryldiimino)-bis-[2,4,6-trijod-isophthalsäure-(2,3-dihydroxy-propyl-N-methyl)-diamid]; Fp. 235–320 °C (Zersetzung).

Beispiel 7

59,5 g 5-Amino-2,4,6-trijod-isophthalsäure-dichlorid werden mit 13,5 g 3,6-Dithiaoctandisäuredichlorid, wie in Beispiel 1 unter A) beschrieben, kondensiert. Man erhält 36,8 g (54% der Theorie) 3,6-Dithiaoctandisäure-bis-[3,5-bis-(chlorcarbonyl)-2,4,6-trijod-anilid] vom Schmelzpunkt 270 °C (Zersetzung). 30 g dieses Tetrachlorids werden, wie in Beispiel 1 unter B) beschrieben, mit 11,5 g N-Methylamino-propandiol-(2,3) umgesetzt. Nach Aufarbeiten durch Fällen in Methylenchlorid, Entfernen der Anionen und Kationen mittels schwach basischen und schwach sauren Ionenaustauschern und Umfällen aus Methanol/Isopropanol

(1:2), erhält man 20 g (55% der Theorie) 3,6-Dithiaoctandisäure-bis-[3,5-bis-(2,3-dihydroxy-propyl-N-methyl-carbamoyl)-2,4,6-trijod-anilid] vom Schmelzpunkt 245 °C (Zersetzung).

Beispiel 8

59,5 g 5-Amino-2,4,6-trijod-isophthalsäure-dichlorid werden mit 15,1 g 3,8-Dithiadecandisäuredichlorid, wie in Beispiel 1 unter A) beschrieben, kondensiert. Man erhält 40,5 g (58% der Theorie) 3,8-Dithiadecandisäure-bis-[3,5-bis-(chlorcarbonyl)-2,4,6-trijod-anilid] vom Schmelzpunkt 245 °C. 30 g dieses Tetrachlorids werden, wie in Beispiel 1 unter B) beschrieben, mit 11,5 g N-Methyl-amino-propandiol-(2,3) umgesetzt. Nach Aufarbeiten durch Fällen in Methylenchlorid, Entfernen der Anionen und Kationen mittels schwach basischen und schwach sauren Ionenaustauschern und Umfällen aus Methanol/Isopropanol (1:2) erhält man 22 g (59% der Theorie) 3,8-Dithiadecandisäure-bis-[3,5-bis-(2,3-dihydroxy-propyl-N-methyl-carbamoyl)-2,4,6-trijod-anilid] vom Schmelzpunkt 250 °C (Zersetzung).

Beispiel 9

59,5 g 5-Amino-2,4,6-trijod-isophthalsäure-dichlorid werden mit 14,4 g 4-Selena-heptandisäuredichlorid, wie in Beispiel 1 unter A) beschrieben, kondensiert. Man erhält 37,5 g (54% der Theorie) 4-Selena-heptan-disäure-bis-[3,5-bis(chlorcarbonyl)-2,4,6-trijod-anilid]; Fp. 246 °C (Zersetzung). 30 g dieses Tetrachlorids werden, wie in Beispiel 1 unter B) beschrieben, mit 11,4 g N-Methylamino-propandiol-(2,3) umgesetzt. Nach Aufarbeiten durch Fällen in Methylenchlorid, Entfernen der Anionen und Kationen mittels schwach basischen und schwach sauren Ionenaustauschern und Umfällen aus Methanol/Isopropanol (1:2) erhält man 16,4 g (46% der Theorie) 4-Selena-heptan-disäure-bis[3,5-bis-(2,3-dihydroxy-propyl-N-methyl-carbamoyl)-2,4,6-trijod-anilid]; Fp. 215–260 °C (Zersetzung).

Beispiel 10

59,5 g 5-Amino-2,4,6-trijod-isophthalsäure-dichlorid werden mit 12,9 g 3,5-Dithiaheptandisäuredichlorid, wie in Beispiel 1 unter A) beschrieben, kondensiert. Man erhält 41,3 g (61% der Theorie) 3,5-Dithiaheptandisäure-bis-[3,5-bis-(chlorcarbonyl)-2,4,6-trijod-anilid] vom Schmelzpunkt 240/250 °C (Zersetzung). 30 g dieses Tetrachlorids werden, wie in Beispiel 1 unter B) beschrieben, mit 11,7 g N-Methylamino-propandiol-(2,3) umgesetzt. Nach Aufarbeiten durch Fällen in Dichlormethan wird das Rohprodukt in Wasser aufgenommen und mit Kohle ausgerührt. Die Rohlösung wird über ein Adsorberharz filtriert, dabei wird die abfliessende wässrige Lösung auf ihren Ionengehalt mittels Messung der Leitfähigkeit geprüft. Die auf dem Harz adsorbierte Substanz wird nach erschöpfendem Waschen mit Wasser mittels Äthanol eluiert. Die so vorgereinigte Substanz wird zur Reinigung über einen schwach basischen und schwach sauren Austauscher filtriert. Nach Umfällen des so isolierten Rohproduktes aus Methanol/Isopropanol (1:2) erhält man 24,4 g (69% der Theorie) 3,5-Dithia-heptandisäure-bis[3,5-bis-(2,3-dihydroxypropyl-N-methyl-carbamoyl)-2,4,6-trijod-anilid]; Fp. 290 °C (Zersetzung).

Beispiel 11

59,5 g 5-Amino-2,4,6-trijod-isophthalsäure-dichlorid werden mit 14,4 g 4,6-Dithianonandisäuredichlorid, wie in Beispiel 1 unter A) beschrieben, kondensiert. Man erhält 37,1 g (53,7% der Theorie) 4,6-Dithia-nonandisäure-bis[3,5-bis(chlorcarbonyl)-2,4,6-trijod-anilid] vom Schmelzpunkt 220–260 °C (Zersetzung). 25 g dieses Tetrachlorids werden, wie in Beispiel 1 unter B) beschrieben, mit 10 g N-Methylamino-propandiol-(2,3) umgesetzt. Nach Aufarbeiten durch Fällen in Methylenchlorid, Entfernen der Anionen und Kationen mittels schwach basischer und schwach saurer Ionenaustauscher und Umfällen des erhaltenen Rohprodukts aus Methanol/Isopropanol (1:2) erhält man 17,3 g (55% der Theorie) 4,6-Dithianonandisäure - bis[3,5 - bis(2,3 - dihydroxypropyl-N-methylcarbamoyl]2,4,6-trijod-anilid]; Fp. 230–260 °C (Zersetzung).

Beispiel 12

4-Thiaheptandisäure-mono-[3,5-bis(2,3-dihydroxypropyl-N-methylcarbamoyl)-2,4,6-trijod-anilid]-mono-[3,5-bis-(2,3-dihydroxypropyl-N-methylcarbamoyl)-2,4,6-trijod-N-methyl-anilid]

A) 115 g 5-Methylamino-2,4,6-trijod-isophthalsäuredichlorid werden bei Raumtemperatur in 80 ml Dimethylacetamid gelöst. Hierzu werden 44,6 g Thiodipropionsäuremonomethylat-monochlorid zugetropft, wobei die Temperatur unter 30 °C gehalten wird. Nach 24 Stunden Rühren wird ausgefallenes Nebenprodukt abgesaugt und das Filtrat im Vakuum eingeengt. Der ölige Rückstand wird mit Methylenchlorid über Silicagel chromatographiert. Man fasst die reine Substanz enthaltenden Fraktionen zusammen, engt die Lösung ein und rührt den Rückstand mit 100 ml trockenem Äther aus. Man erhält 71 g (47% der Theorie) 2,4,6-Trijod-3[N-methyl-N-(methoxy-4-thia-pimeloyl)amino]-isophthalsäuredichlorid vom Schmelzpunkt 153 °C. 31,2 g dieses Halbesters werden in 300 ml eines Dioxan/Wasser-Gemisches 1:1 suspendiert und bei 80 °C mit 1 Äquivalent (120 mMol) Natronlauge versetzt. Nach 4 Stunden lässt man abkühlen, rührt über Nacht nach und engt im Vakuum ein. Der Rückstand wird in wenig Methanol aufgenommen und in vorgelegtes Aceton eingerührt. Der erhaltene Niederschlag wird abgesaugt und 3 Stunden im Vakuum bei 50 °C getrocknet. Man erhält 17,2 g (54% der Theorie) 2,4,6-Trijod-3[N-methyl-N-(hydroxy-4-thia-pimeloyl)amino]isophthalsäure als Trinatriumsalz vom Schmelzpunkt 300 °C (Zersetzung). 15 g dieser Trisäure werden in 150 ml Toluol suspendiert und vorhandenes Wasser azeotrop abdestilliert. Bei 10 °C werden 17,9 g Phosphorpentachlorid eingetragen und zwei Stunden gerührt. Die Reaktionslösung wird dann im Vakuum bei ca. 10 °C eingeengt. Man erhält 17,5 g (108% der Theorie) rohes 2,4,6-Trijod-3[N-methyl-N-(chlor-4-thia-pimeloyl)-amino]-isophthalsäuredichlorid. 17 g dieses rohen, feuchtigkeitsempfindlichen Trisäurechlorids werden unter Stickstoff portionenweise in eine Lösung von 12,8 g 5-Amino-2,4,6-trijod-isophthalsäuredichlorid in 25 ml Dimethylacetamid gegeben. Man rührt 16 Stunden bei 30 °C und saugt das ausgefallene Tetrachlorid ab. Das Rohprodukt wird in 150 ml Trichlormethan gelöst und über Silicagel filtriert. Man erhält 15,3 g 4-Thiaheptandisäuremono-[3,5-bis(chlorcarbo-nyl)-2,4,6-trijod-N-methylanilid]-mono[3,5-bis-(chlorcarbonyl)-2,4,6-trijod-anilid] («Tetrachlorid») vom Schmelzpunkt 240–260 °C (Zersetzung).

B) Amidierung

15 g des Tetrachlorids werden in 21 g auf 50–60 °C erwärmtes, mit Stickstoff begastes N-Methyl-propandiol-(2,3) eingetragen. Das Reaktionsgemisch wird 4 Stunden bei dieser Temperatur stehengelassen und dann mit 25 ml trockenem Dioxan versetzt. Man dekantiert das Dioxan, nimmt den Rückstand mit 50 ml Methanol auf und fällt mit 250 ml Isopropanol. Das ausgefallene Rohprodukt wird abgesaugt und analog Beispiel 1 von Ionen befreit. Man erhält 11,2 g 4-Thiaheptandisäure-mono-[3,5-bis-(2,3-dihydroxy-propyl-N-methyl-carbamoyl)-2,4,6-trijod-anilid]mono-[3,5-bis-(2,3-dihydroxypropyl-N-methyl-carbamoyl)-2,4,6trijod-N-methyl-anilid] vom Schmelzpunkt 250 °C (Zersetzung).

Beispiel 13

20 g Thiodipropionsäure-bis-[3,5-bis(chlor-carbonyl)-2,4,6-trijod-anilid] werden in 50 ml Dimethylformamid gelöst und bei Zimmertemperatur 29,2 g Methylglucamin, suspendiert in 50 ml Dimethylformamid, zugetropft. Dabei tritt Wärmetönung ein. Man rührt 4 Stunden bei 50 °C, lässt abkühlen und rührt das Reaktionsgemisch in 500 ml Isopropanol ein. Die Ausfällung wird abgesaugt, in Methanol/Dimethylformamid aufgenommen und, wie für Beispiel 1 beschrieben, aufgereinigt. Man erhält 21 g (70% der Theorie) Thiodipropionsäure-bis-[3,5-bis-(2,3,4,5,6-pentahydroxyhexyl-N-methyl-carbamoyl)-2,4,6-trijod-anilid], Schmelzpunkt: 230 °C (Zersetzung).

Beispiel 14

59,5 g 5-Amino-2,4,6-trijod-isophthalsäure-dichlorid werden mit 12,7 g 3-Oxa-6-thiaoctandisäuredichlorid, wie in Beispiel 1 unter A) beschrieben, kondensiert. Man erhält 34 g (50,6% der Theorie) 3-Oxa-6-thiaoctandisäure-bis-[3,5-bis-(chlorcarbonyl)-2,4,6-trijod-anilid], Schmelzpunkt 272–290 °C (Zersetzung). 23,7 g dieses Tetrachlorids werden, wie in Beispiel 1 unter B) beschrieben, mit 7,8 g N-Methyl-amino-propandiol-(2,3) umgesetzt. Die Reaktionslösung wird in einen Liter Isopropanol eingerührt. Die ausgefallene Substanz wird abgesaugt, mit Isopropanol gewaschen und getrocknet. Die Rohsubstanz wird in Methanol gelöst und, wie für Beispiel 1 beschrieben, durch Filtration über Ionenaustauscher und Umfällen aus Methanol/Isopropanol (1:2) gereinigt. Man erhält 11,7 g (49% der Theorie) 3-Oxa-6-thia-octandisäure-bis-[3,5-bis-(2,3-dihydroxypropyl-N-methyl-carbamoyl)-2,4,6-trijod-anilid]; Schmelzpunkt 285–290 °C (Zersetzung).

Beispiel 15

4,4,6,6-Tetramethyl-4,6-disila-5-oxa-nonandisäure-bis[3,5-bis(2,3-dihydroxypropyl-N-methylcarbamoyl)-2,4,6-trijodanilid]

A) 4,4,6,6-Tetramethyl-4,6-disila-5-oxa-nonandisäure-bis[3,5-bis-(chlorcarbonyl)-2,4,6-trijod-anilid] («Tetrachlorid»)

19,4 g 5-Amino-2,4,6-trijod-isophthalsäuredichlorid werden heiss in 20 ml Dioxan gelöst und zu dieser Lösung innerhalb von 10 Minuten 6,2 g 4,4,6,6-Tetramethyl-4,6-disila-5-oxanonandisäuredichlorid unter Sieden zugetropft. Man hält die Mischung 4 Stunden am Rückfluss und lässt dann über Nacht bei Raumtemperatur stehen. Danach wird abgesaugt, der Filterrückstand mit wenig kaltem Dioxan nachgewaschen und aus Benzol umkristallisiert.

Man erhält 9,25 g «Tetrachlorid» (4,4,6,6-Tetramethyl-4,6-disila-5-oxanonandisäure-bis-[3,5-bischlorocarbonyl-2,4,6-trijod-anilid], Schmelzpunkt: 212–213 °C; Ausbeute 40% der Theorie.

B) 7 g «Tetrachlorid» werden zu einer Lösung von 6,14 g N-Methylaminopropandiol-(2,3) in 25 ml Dimethylacetamid (DMA) bei 40 °C zugetropft. Man hält die Lösung danach 2 Stunden bei 50 °C und rührt in 250 ml Isopropyläther ein. Der ölige Niederschlag wird in wenig Methanol gelöst, wiederum in Isopropyläther gefällt, um restliches DMA zu entfernen, und der Niederschlag abfiltriert. Zur weiteren Reinigung löst man den Rückstand in 350 ml Methanol und filtriert die Lösung zunächst über einen schwach basischen Anionenaustauscher (Lewatit MP 7080) und danach über einen schwach sauren Kationenaustauscher (Lewatit CP 3050). Nach dem Eindampfen erhält man 7,8 g 4,4,6,6-Tetramethyl-4,6-disila-5-oxa-nonandisäure-bis[3,5-bis(2,3-dihydroxypropyl-N-methylcarbamoyl)-2,4,6-trijod-anilid] (93% der Theorie), Zersetzungspunkt 220 °C.

Beispiel 16

5,5-Dimethyl-5-sila-nonandisäure-bis[3,5-bis-(2,3-dihydroxypropyl-N-methyl-carbamoyl)-2,4,6-trijodanilid]

A) 5,5-Dimethyl-5-sila-nonandisäure-bis[3,5-bis(chlorcarbonyl)-2,4,6-trijod-anilid] («Tetrachlorid»)

8,4 g 5-Amino-2,4,6-trijod-isophthalsäuredichlorid werden durch Erwärmen in 9 ml Dioxan gelöst und 4,5 g 5,5-Dimethyl-5-sila-nonandisäuredichlorid innerhalb von 10 Minuten unter Sieden zugetropft. Man hält die Mischung noch 3,5 Stunden am Rückfluss und lässt über Nacht bei Raumtemperatur stehen. Danach wird abgesaugt, der Filterrückstand mit wenig kaltem Dioxan gewaschen und die Testsubstanz aus Benzol umkristallisiert. Man erhält 4,76 g «Tetrachlorid» (49% der Theorie); Schmelzpunkt: 225–230 °C (Zersetzung).

B) Aus 4,75 g «Tetrachlorid» und 4,3 g N-Methylamino-propandiol-(2,3) werden, wie in Beispiel 1 unter B) beschrieben, 5,1 g 5,5-Dimethyl-5-

sila-nonandisäure-bis[3,5-bis(2,3-dihydroxypropyl-N-methyl-carbamoyl)-2,4,6-trijod-anilid] erhalten (90% der Theorie); Zersetzungspunkt: 225 °C.

Beispiel 17
4,4-Dimethyl-4-sila-heptandisäure-bis[3,5-bis-(1,3-dihydroxypropylcarbamoyl)-2,4,6-trijod-anilid]

A) 4,4-Dimethyl-4-sila-heptandisäure-bis[3,5-bis(chlorcarbamoyl)-2,4,6-trijod-anilid] («Tetrachlorid»)

6,3 g 5-Amino-2,4,6-trijod-isophthalsäuredichlorid und 1,5 g 4,4-Dimethyl-4-sila-heptandisäure-dichlorid werden, wie in Beispiel 2 unter A) beschrieben, umgesetzt zu 2,9 g «Tetrachlorid» (40% der Theorie); Zersetzungspunkt ca. 215 °C.

B) Das erhaltene «Tetrachlorid» (2,9 g) wird, wie in Beispiel 1 unter B) beschrieben, mit 2,3 g 2-Aminopropan-diol-(1,3) umgesetzt zu 2,69 g 4,4-Dimethyl-4-sila-heptandisäure-bis[3,5-bis(1,3-dihydroxypropyl-carbamoyl)-2,4,6-trijod-anilid], (80% der Theorie): Zersetzungspunkt: 230 °C.

Beispiel 18
Herstellung einer gebrauchsfertigen Röntgenkontrastmittel-Lösung für die Lymphographie

Die Lösung von 61,68 g 4,4,6,6-Tetramethyl-4,6-disila-5-oxa-nonandisäure-bis[3,5-bis(2,3-dihydroxypropyl-N-methyl-carbamoyl)-2,4,6-trijod-anilid], von 10 mg wasserfreiem Calcium-dinatrium-edetat und von 4,0 g 1,2-Propandiol in 90 ml bidestilliertem Wasser wird durch Zugabe von 0,1 n Natronlauge auf einen pH-Wert von 7,2 eingestellt und schliesslich mit bidestilliertem Wasser auf 100 ml aufgefüllt. Diese Lösung wird in Ampullen oder Multivials abgefüllt und 20 Minuten bei 120 °C sterilisiert. Diese Lösung enthält 275 mg Jod/ml.

Beispiel 19
Herstellung einer gebrauchsfertigen Röntgenkontrastmittel-Lösung für die Lymphographie

Man löst 58,09 g 3,3'-Thiodipropionsäure-bis-[3,5-bis(2,3-dihydroxypropyl-N-methyl-carbamoyl)-2,4,6-trijod-anilid], 10 mg wasserfreies Calcium-dinatrium-edetat und 4,0 g 1,2-Propandiol in 90 ml bidestilliertem Wasser, stellt den pH-Wert dieser Lösung durch Zugabe von 0,1 n Natronlauge auf 7,2 ein und fällt schliesslich mit bidestilliertem Wasser auf 100 ml auf. Die Lösung wird durch ein Porenfilter mit einer Porentiefe von 0,2 µm gegeben, in Ampullen oder Multivials abgefüllt und 20 Minuten bei 120 °C sterilisiert.

Die Lösung enthält 275 mg Jod/ml.

**Patentansprüche**

1. Trijodierte 5-Aminoisophthalsäure-Derivate der allgemeinen Formel I

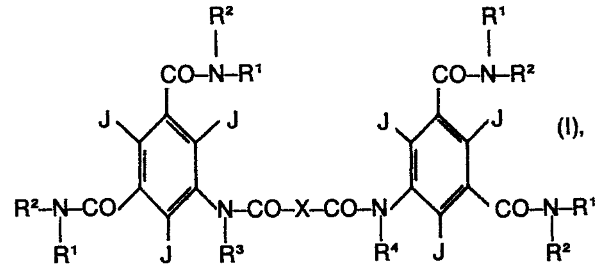
(I),

worin

$R^1$ einen gerad- oder verzweigtkettigen Mono- oder Polyhydroxyalkylrest mit 2–8 Kohlenstoffatomen und 1–5 Hydroxygruppen,

$R^2$ Wasserstoff, einen Alkylrest mit 1–4 Kohlenstoffatomen oder $R^1$,

$R^3$ und $R^4$ gleich oder verschieden sind und Wasserstoff oder einen Alkylrest mit 1–4 Kohlenstoffatomen bedeuten und

X ein gerad- oder verzweigtkettiges Alkylen mit 2–12 Kohlenstoffatomen, das durch 1 bis 3 Schwefel- oder 1 bis 2 Selenatome und gegebenenfalls zusätzlich durch ein Sauerstoffatom oder durch eine Di-($C_1$–$C_4$-alkyl)-silylgruppe oder eine Tetra-($C_1$–$C_4$-alkyl)-disiloxangruppe unterbrochen ist, darstellt.

2. 3,3'-Thiodipropionsäure-bis-[3,5-bis-(2,3-dihydroxypropyl-N-methyl-carbamoyl)-2,4,6-trijod-anilid]

3. 3,6-Dithiaoctandisäure-bis-[3,5-bis-(2,3-dihydroxypropyl-N-methyl-carbamoyl)-2,4,6-trijod-anilid]

4. 4,4,6,6-Tetramethyl-4,6-disila-5-oxa-nonandisäure-bis-[3,5-bis-(2,3-dihydroxypropyl-N-methylcarbamoyl)-2,4,6-trijod-anilid]

5. Verfahren zur Herstellung trijodierter 5-Amino-isophthalsäure-Derivate der allgemeinen Formel I, dadurch gekennzeichnet, dass man in an sich bekannter Weise ein Tetracarbonsäuretetrachlorid der allgemeinen Formel II

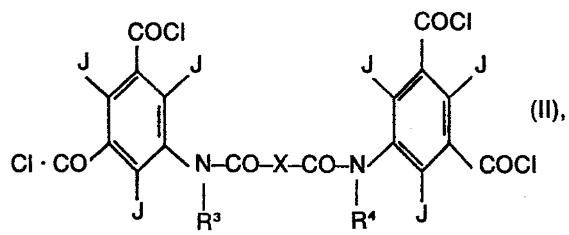
(II),

worin $R^3$, $R^4$ und X die in Anspruch 1 angegebene Bedeutung haben, mit einem Amin 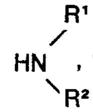, worin $R^1$ und $R^2$ die in Anspruch 1 angegebene Bedeutung haben, umsetzt.

6. Röntgenkontrastmittel enthaltend mindestens eine der Verbindungen gemäss Ansprüchen 1 bis 4.

7. Trijodierte 5-Aminoisophthalsäure-tetrachloride der allgemeinen Formel II, worin $R^3$, $R^4$ und X die obengenannte Bedeutung haben.

## Claims

1. Triiodised 5-aminoisophthalic acid derivatives of the general formula I

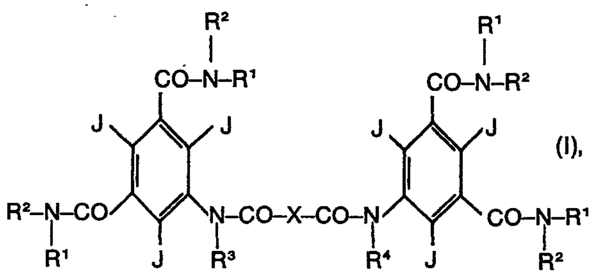

(I),

in which

$R^1$ is a straight-chain or branched mono- or polyhydroxyalkyl radical having from 2 to 8 carbon atoms and from 1 to 5 hydroxy groups,

$R^2$ represents hydrogen, an alkyl radical having from 1 to 4 carbon atoms or $R^1$,

$R^3$ and $R^4$ are the same or different and represent hydrogen or an alkyl radical having from 1 to 4 carbon atoms, and

X represents a straight-chain or branched alkylene having from 2 to 12 carbon atoms that is interrupted by from 1 to 3 sulphur atoms or by 1 or 2 selenium atoms and, optionally, additionally by an oxygen atom or by a di-($C_1$–$C_4$-alkyl)-silyl group or a tetra-($C_1$–$C_4$-alkyl)-disiloxane group.

2. 3,3'-thiodipropionic acid bis-[3,5-bis-(2,3-dihydroxypropyl-N-methylcarbamoyl)-2,4,6-triiodoanilide].

3. 3,6-dithiaoctanedioic acid bis-[3,5-bis-(2,3-dihydroxypropyl-N-methylcarbamoyl)-2,4,6-triodoanilide].

4. 4,4,6,6-tetramethyl-4,6-disila-5-oxa-nonadioic acid bis-[3,5-bis-(2,3-dihydroxypropyl-N-methylcarbamoyl)-2,4,6-triiodoanilide].

5. Process for the manufacture of triiodised 5-aminoisophthalic acid derivatives of the general formula I, characterised in that, in a manner known per se, a tetracarboxylic acid tetrachloride of the general formula II

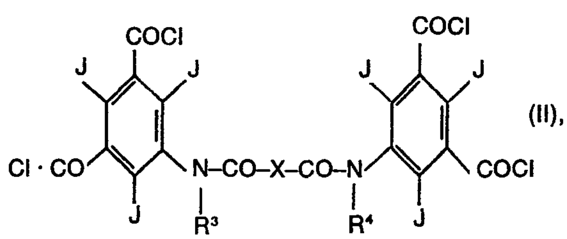

(II),

in which $R^3$, $R^4$ and X have the meanings given in claim 1 is reacted with an amine 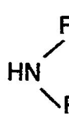 in which $R^1$ and $R^2$ have the meanings given in claim 1.

6. X-ray contrast agents containing at least one of the compounds according to claims 1 to 4.

7. Triiodised 5-aminoisophthalic acid tetrachlorides of the general formula II, in which $R^3$, $R^4$ and X have the meanings given above.

## Revendications

1. Dérivés d'acides amino-5 isophthaliques triiodés répondant à la formule générale (I):

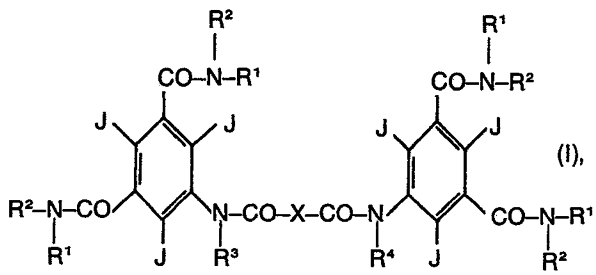

(I),

dans laquelle:

$R^1$ représente un radical monohydroxy- ou polyhydroxy-alkyle, linéaire ou ramifié, contenant de 2 à 8 atomes de carbone et portant de 1 à 5 groupes hydroxy,

$R^2$ représente l'hydrogène, un radical alkyle contenant de 1 à 4 atomes de carbone ou un radical tel que défini pour $R^1$,

$R^3$ et $R^4$ sont identiques ou différents et représentent chacun l'hydrogène ou un radical alkyle contenant de 1 à 4 atomes de carbone, et

X représente un radical alkylène en $C_2$–$C_{12}$, linéaire ou ramifié, qui est interrompu par 1 à 3 atomes de soufre ou par 1 ou 2 atomes de sélénium et éventuellement, en plus, par 1 atome d'oxygène, ou par un radical bis-($C_1$–$C_4$-alkyl)-silyle ou un radical de tétrakis-($C_1$–$C_4$-alkyl)-disiloxane.

2. Bis-[bis-(N-dihydroxy-2,3 propyl)-N-méthylcarbamoyl)-3,5 triiodo-2,4,6 anilide] de l'acide thio-3,3' dipropionique.

3. Bis-[bis-(N-(dihydroxy-2,3 propyl)-N-méthylcarbamoyl)-3,5 triiodo-2,4,6 anilide] de l'acide dithia-3,6 octanedioïque.

4. Bis-[bis-(N-(dihydroxy-2,3 propyl)-N-méthylcarbamoyl)-3,5 triiodo-2,4,6 anilide] de l'acide tétraméthyl-4,4,6,6 disila-4,6 oxa-5 nonanedioïque.

5. Procédé de préparation de dérivés d'acides amino-5 isophthaliques triiodés répondant à la formule générale (I), procédé caractérisé en ce qu'on fait réagir, de manière connue, un tétrachlorure d'acide tétracarboxylique répondant à la formule générale (II):

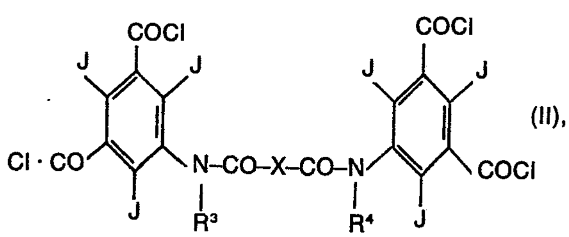

(II),

dans laquelle $R^3$, $R^4$ et X ont les significations données dans la revendication 1, avec une amine

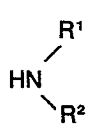

dans laquelle $R^1$ et $R^2$ ont les significations données à la revendication 1.

6. Agent de contraste pour rayons X qui renferme au moins un des composés selon l'une quelconque des revendications 1 à 4.

7. Tétrachlorures d'acides amino-5 isophthaliques triiodés répondant à la formule générale (II) dans laquelle $R^3$, $R^4$ et X ont les significations indiquées ci-dessus.